# EUROPEAN PATENT SPECIFICATION

(11) **EP 2 558 096 B1**
(45) Date of publication and mention of the grant of the patent: **05.06.2019**
(21) Application number: 11715912.9
(22) Date of filing: 15.04.2011
(51) Int. Cl.: A61K 31/497, A61K 31/74, A61K 45/06, A61P 35/00

(54) **COMBINATION OF HYPOXIA-INDUCIBLE FACTOR-1 INHIBITORS AND TOLL-LIKE RECEPTOR-3 AGONISTS FOR TREATING SOLID TUMORS**
KOMBINATION AUS HYPOXIE-INDUZIERBAREM FAKTOR-1-HEMMERN UND TOLL-LIKE-REZEPTOR-3-AGONISTEN ZUR BEHANDLUNG VON SOLIDEN TUMOREM
COMBINAISON D'INHIBITEURS DU FACTEUR INDUCTIBLE PAR L'HYPOXIE HIF-1 (HIF-1) ET D'AGONISTES DES RÉCEPTEURS TOLL-LIKE 3 (TLR-3) POUR TRAITER DES TUMEURS SOLIDES

(30) Priority: 16.04.2010 IT RM20100185
(43) Date of publication of application: 20.02.2013
(73) Proprietor: Università degli Studi di Roma "La Sapienza", 00185 Roma (IT)
(72) Inventor: FILIPPINI, Antonio, I-00185 Roma (IT); RICCIOLI, Anna, I-00185 Roma (IT); PAONE, Alessio, I-00185 Roma (IT); ZIPARO, Elio, I-00185 Roma (IT)
(74) Representative: Capasso, Olga
(86) International application number: PCT/EP2011/056006
(87) International publication number: WO 2011/128436

(56) References cited:
- WO-A1-2009/112851
- ONNIS BARBARA ET AL: "Development of HIF-1 inhibitors for cancer therapy.", JOURNAL OF CELLULAR AND MOLECULAR MEDICINE SEP 2009 LNKD- PUBMED:19674190, vol. 13, no. 9A, September 2009 (2009-09), pages 2780-2786, XP002608195, ISSN: 1582-4934 cited in the application
- GIOVANNI MELILLO: "Targeting hypoxia cell signaling for cancer therapy", CANCER AND METASTASIS REVIEWS, KLUWER ACADEMIC PUBLISHERS, DO LNKD- DOI:10.1007/S10555-007-9059-X, vol. 26, no. 2, 6 April 2007 (2007-04-06), pages 341-352, XP019524649, ISSN: 1573-7233
- JASANI B ET AL: "Ampligen: A potential toll-like 3 receptor adjuvant for immunotherapy of cancer", VACCINE, ELSEVIER LTD, GB LNKD- DOI:10.1016/J.VACCINE.2009.01.071, vol. 27, no. 25-26, 26 May 2009 (2009-05-26), pages 3401-3404, XP026122472, ISSN: 0264-410X [retrieved on 2009-02-05] cited in the application
- PAONE ALESSIO ET AL: "Toll-like receptor 3 triggers apoptosis of human prostate cancer cells through a PKC-alpha-dependent mechanism.", CARCINOGENESIS JUL 2008 LNKD- PUBMED:18566014, vol. 29, no. 7, 1 July 2008 (2008-07-01), pages 1334-1342, XP002608197, ISSN: 1460-2180
- PAONE ALESSIO ET AL: "Toll-like receptor 3 regulates angiogenesis and apoptosis in prostate cancer cell lines through hypoxia-inducible factor 1 alpha.", NEOPLASIA (NEW YORK, N.Y.) JUL 2010 LNKD- PUBMED:20651983, vol. 12, no. 7, July 2010 (2010-07), pages 539-549, XP009140777, ISSN: 1476-5586

## Description

### FIELD OF THE INVENTION

The present invention relates to a combination of a HIF-1 inhibitor and a TLR-3 agonist. The combination is for use as an anti-tumoral agent of a solid tumor, preferably prostate tumor.

### BACKGROUND ART

Hypoxia inducible factor 1 (HIF-1) is a basic helix-loop-helix transcription factor, which regulates a number of genes required for hypoxic response via binding specific regions of their promoters, named hypoxia responsive elements (HREs) [1-3]. HIF-1 is active only as a heterodimer of HIF-1α and HIF-1β subunits. HIF-1β is constitutively expressed in all cell types, whereas HIF-1α levels are tightly controlled. Under normoxia HIF-1α levels are low, due to proteasomal degradation initiated by oxygen-sensing prolyl hydroxylases (PHDs). Under hypoxic conditions, HIF-1α is stabilized and freely binds HIF-1β forming active HIF-1 transcription complex [4]. In humans three different isoform of *hif-1α* were recently described: 1.1 ubiquitous and responsible for the transcriptional activity of the hypoxic response; 1.2 expressed specifically in the testis plays a dominant negative function with respect to 1.1 isoform [5]; 1.3 isoform has recently been found highly expressed in peripheral blood leukocytes, in the thymus and in activated-T-cells [6].

It has been clearly demonstrated that HIF-1 regulates genes particularly relevant to cancer progression (reviewed in [7]), especially as predictor of clinical outcome in patients with adenocarcinoma [7b]. In particular, HIF-1α has emerged as a potential prognostic biomarker in proteomic assessment of prostate cancer [8] since clinical observation of high-grade prostate intraepithelial neoplasia (PIN) lesion (precursor of most prostate adenocarcinoma (PCa)), showed increased HIF-1α expression [9] and HIF-1α up-regulation in PCa as well as in prostate cancer bone metastases has been observed [10]. PCa is a prevalent tumour among elderly men and survival benefit with current PCa therapies is often limited [11]. Indeed standard pharmacological therapy, consisting of withdrawal of androgens, leads to only transient regression of the disease and there is no cure for prostate cancer once it becomes androgen refractory. Although HIF-1α protein is mainly induced by hypoxic conditions, other stimuli can strongly increase the HIF-1 complex in normoxic conditions and modulate the transcription of hypoxic genes. These stimuli include reactive nitrogen-(RNS) [12] or oxygen-derived radicals (ROS) [13], cytokines [14,15], growth factors [16] and T-cell receptor (TCR) stimulation [6,17]. Interestingly, also a variety of molecular components derived from bacteria or viruses has been described to activate HIF-1α in normoxia through specific Toll Like Receptors (TLRs) [18-20]. These are a group of transmembrane proteins (11 in humans) that recognize pathogen-associated molecular patterns (PAMPS) and elicit antimicrobial immune responses [21]. TLRs were initially detected only on immune cells [22], but recent studies demonstrate that tumour cells express functional TLRs and that TLR signaling can promote opposite outcomes: tumour growth and immune evasion, or apoptosis and cell cycle arrest [23-26].

Toll like Receptor-3 (TLR3) is a member of the Toll-like receptor (TLR) family which plays a fundamental role in pathogen recognition and activation of innate immunity. TLRs are highly conserved from Drosophila to humans and share structural and functional similarities. The various TLRs exhibit different patterns of expression. This receptor is most abundantly expressed in placenta and pancreas, and is restricted to the dendritic subpopulation of the leukocytes. It recognizes dsRNA associated with viral infection, and induces the activation of NF-kappaB and the production of type I interferons. It may thus play a role in host defence against viruses. TLR3 mRNA sequence is described in NCBI accession number NM_003265, the sequence of which is shown in SEQ ID NO: 1.

cactttcgag agtgccgtct atttgccaca cacttccctg atgaaatgtc
tggatttggactaaagaaaa aaggaaaggc tagcagtcat ccaacagaat catgagacag
actttgcctt gtatctactt ttgggggggc cttttgccct ttgggatgct gtgtgcatcc
tccaccacca agtgcactgt tagccatgaa gttgctgact gcagccacct gaagttgact
caggtacccg atgatctacc cacaaacata acagtgttga accttaccca taatcaactc
agaagattac cagccgccaa cttcacaagg tatagccagc taactagctt ggatgtagga
tttaacacca tctcaaaact ggagccagaa ttgtgccaga aacttcccat gttaaaagtt
ttgaacctcc agcacaatga gctatctcaa ctttctgata aaacctttgc cttctgcacg
aatttgactg aactccatct catgtccaac tcaatccaga aaattaaaaa taatcccttt
gtcaagcaga agaatttaat cacattagat ctgtctcata atggcttgtc atctacaaaa
ttaggaactc aggttcagct ggaaaatctc caagagcttc tattatcaaa caataaaatt
caagcgctaa aaagtgaaga actggatatc tttgccaatt catctttaaa aaaattagag
ttgtcatcga atcaaattaa agagttttct ccagggtgtt ttcacgcaat tggaagatta
tttggcctct ttctgaacaa tgtccagctg ggtcccagcc ttacagagaa gctatgtttg
gaattagcaa acacaagcat tcggaatctg tctctgagta acagccagct gtccaccacc
agcaatacaa ctttcttggg actaaagtgg acaaatctca ctatgctcga tctttcctac
aacaacttaa atgtggttgg taacgattcc tttgcttggc ttccacaact agaatatttc

ttcctagagt ataataatat acagcatttg ttttctcact ctttgcacgg gcttttcaat
gtgaggtacc tgaatttgaa acggtctttt actaaacaaa gtatttccct tgcctcactc
cccaagattg atgatttttc ttttcagtgg ctaaaatgtt tggagcacct taacatggaa
gataatgata ttccaggcat aaaaagcaat atgttcacag gattgataaa cctgaaatac
ttaagtctat ccaactcctt tacaagtttg cgaactttga caaatgaaac atttgtatca
cttgctcatt ctcccttaca catactcaac ctaaccaaga ataaaatctc aaaaatagag
agtgatgctt tctcttggtt gggccaccta gaagtacttg acctgggcct taatgaaatt
gggcaagaac tcacaggcca ggaatggaga ggtctagaaa atattttcga aatctatctt
tcctacaaca agtacctgca gctgactagg aactcctttg ccttggtccc aagccttcaa
cgactgatgc tccgaagggt ggcccttaaa aatgtggata gctctccttc accattccag
cctcttcgta acttgaccat tctggatcta agcaacaaca acatagccaa cataaatgat
gacatgttgg agggtcttga gaaactagaa attctcgatt tgcagcataa caacttagca
cggctctgga aacacgcaaa ccctggtggt cccatttatt tcctaaaggg tctgtctcac
ctccacatcc ttaacttgga gtccaacggc tttgacgaga tcccagttga ggtcttcaag
gatttatttg aactaaagat catcgattta ggattgaata atttaaacac acttccagca
tctgtcttta ataatcaggt gtctctaaag tcattgaacc ttcagaagaa tctcataaca
tccgttgaga agaaggtttt cgggccagct ttcaggaacc tgactgagtt agatatgcgc
tttaatccct ttgattgcac gtgtgaaagt attgcctggt ttgttaattg gattaacgag
acccatacca acatccctga gctgtcaagc cactaccttt gcaacactcc acctcactat
catgggttcc cagtgagact tttgatacat catcttgca aagacagtgc cccctttgaa
ctctttttca tgatcaatac cagtatcctg ttgattttta tctttattgt acttctcatc
cactttgagg gctggaggat atctttttat tggaatgttt cagtacatcg agttcttggt
ttcaaagaaa tagacagaca gacagaacag tttgaatatg cagcatatat aattcatgcc
tataaagata aggattgggt ctgggaacat ttctcttcaa tggaaaagga agaccaatct
ctcaaatttt gtctggaaga aagggacttt gaggcgggtg tttttgaact agaagcaatt
gttaacagca tcaaaagaag cagaaaaatt atttttgtta taacacacca tctattaaaa
gacccattat gcaaaagatt caaggtacat catgcagttc aacaagctat tgaacaaaat
ctggattcca ttatattggt tttccttgag gagattccag attataaact gaaccatgca
ctctgtttgc gaagaggaat gtttaaatct cactgcatct tgaactggcc agttcagaaa
gaacggatag gtgcctttcg tcataaattg caagtagcac ttggatccaa aaactctgta
cattaaattt atttaaatat tcaattagca aaggagaaac tttctcaatt taaaaagttc
tatggcaaat ttaagttttc cataaaggtg ttataatttg tttattcata tttgtaaatg
attatattct atcacaatta catctcttct aggaaaatgt gtctccttat ttcaggccta
tttttgacaa ttgacttaat tttacccaaa ataaaacata taagcacgta aaaaaaaaaa
aaaaaaa

TLR3 is described in WO 98/50547.

It has also been found that certain classes of patients with cancer treated in accordance with the procedure described using a TLR3 agonist exhibit greater survival than other patients. One class of patients in which enhanced survival was found were patients having tumors that express a TLR3 protein. Determining whether tumor types express TLR3 can be carried out as described in the examples, e.g. by detecting the presence on one or more TLR3 polypeptides in a biological sample from a cancer patient, generally from a tumor biopsy.

Hypoxia is a major hindrance to effective solid tumor therapy. The microenvironment of rapidly growing solid tumor is associated with increased energy demand and diminished vascular supply, resulting in focal areas of prominent hypoxia, regions with reduced oxygen tensions. Tissue oxygen electrode measurements taken in cancer patients showed a median range of oxygen partial pressure of 10 to 30 mmHg, with a significant proportion of readings below 2.5 mmHg, whereas those in normal tissues ranged from 24 to 66. Hypoxia causes tumor cells to stop or slow their rate of progression through the cell cycle. Because most anticancer drugs are most effective against rapidly proliferating cells than slowly or non-proliferating cells, this slowing of cell proliferation leads to decreased cell killing.

Tumor hypoxia increases malignant progression and metastasis by promoting angiogenesis through induction of both pro-angiogenic proteins such as VEGF, metabolic adaptation through elevation of glycolytic enzymes and anti-apoptotic proteins. Hypoxia also generates selective pressure for cells to acquire genetic alternations that eventually will circumvent hypoxia-induced apoptosis. VEGF, the most important known regulator of tumor angiogenesis is transcriptionally upregulated by HIF-1. In addition to promoting angiogenesis, hypoxic tumor cells are also refractive to radio- and chemo-therapies. Therefore, hypoxic areas of prostate cancer represent an important target for anti-tumor therapy.

HIF is the primary transcription factor activated by hypoxia. Its activation and function are complex, with numerous point of potential inhibition. HIF controls the expression of more than 60 target genes whose products are critical to many aspects of tumor progression, including metabolic adaptation, apoptosis resistance, angiogenesis and metastasis. HIF-1 has been recognized as an important molecular target for solid tumor therapy due to its fundamental role in tumor angiogenesis and progression. Accordingly, there is a need for novel and effective treatments for solid tumors, in particular prostate or breast cancer. In particular, there is a need for new and effective pharmacological strategy that addresses hypoxia and apoptosis inducer such as TLR-3 agonists.

The TLR3 agonist, poly(I:C), mimics the action of double stranded RNA (dsRNA), the genetic material of many viruses, and TLR3 engagement, directly inhibits cell proliferation and induces tumour cell death [26,27]. The authors have previously demonstrated that LNCaP cells, an androgen-dependent human prostate cancer cell line, are sensitive to poly(I:C)-induced apoptosis, whereas PC3 cells, a more aggressive androgen-independent prostate cancer cell line, show a weak sensitivity to the same stimulus [25].

The international application WO 2009/112851 discloses the use of HIF-1 inhibitors such as echinomycin and also TLR-3 agonists such as Ampligen as anticancer agents. The compounds are not disclosed in combination.

Onnis et al. [50] and Melillo et al. [51] disclose the use of HIF-1 inhibitors for the treatment of solid tumors. Jasani et al. [39] discloses the use of TLR3 agonists, in particular poly(I:C12U) in the treatment of cancer.

Recently, a number of articles reported the ability of HIF-1 complex to mediate the resistance to several apoptotic stimuli by inducing anti-apoptotic genes such as Bcl-xL, survivin and MCL-1 described to be HIF-1 target genes [28-30]. Based on these data, the authors have hypothesized that the limited response of PC3 cells to poly(I:C) could be due to the induction of a parallel pro-tumoral signal involving HIF-1 complex activation. In the present invention, the authors report evidence showing that poly(I:C)-treatment activates TLR3 and enhances the transcription of the 1.3 isoform of *hif-1α* in the prostate cancer cell line PC3, but not in the less aggressive LNCaP cells. The authors also demonstrate that TLR3 stimulation of PC3 cells induces HIF-1α-dependent VEGF secretion and resistance to poly(I:C)-induced apoptosis, while these responses to poly(I:C) are obtained in LNCaP cells only when forcing over-expression *hif-1α-* 1.3 isoform. Taken together, the present invention shows that the levels of HIF-1α in prostate cancer cells play a crucial role in the anti-tumour potential of TLR3 stimulation.

### DESCRIPTION OF THE INVENTION

Toll-like receptors (TLRs) recognize microbial/viral-derived components which trigger innate immune response and conflicting data implicate TLR agonists in cancer, either as pro-tumour or anti-tumour agents. The authors previously demonstrated that TLR3 activation mediated by its agonist poly(I:C) induces anti-tumour signaling leading to apoptosis of prostate cancer cells LNCaP and PC3 with much more efficiency in the former than in the second more aggressive line. The transcription factor Hypoxia inducible factor-1 (HIF-1) regulates several cellular processes, including apoptosis, in response to hypoxia and to other stimuli also in normoxic conditions. In the present invention, the authors describe a novel pro-tumour machinery triggered by TLR3 activation in PC3 cells consisting in increased expression of the specific 1.3 isoform of HIF-1α and nuclear accumulation of HIF-1 complex in normoxia, resulting in reduced apoptosis and in secretion of functional VEGF. Moreover, the authors report that in the less aggressive LNCaP cells, TLR3 activation fails to induce nuclear accumulation of HIF-1α. However, the transfection of 1.3 isoform of *hif-1α* in LNCaP cells allows poly(I:C)-induced HIF-1 activation, resulting in apoptosis protection and VEGF secretion. Altogether, the authors findings demonstrate that differences in basal level of HIF-1α expression in different prostate cancer cell lines underlie their differential response to TLR3 activation, suggesting a correlation between different stages of malignancy, hypoxic gene expression and beneficial responsiveness to TLR agonists.

It is an object of the invention a combination of a HIF-1 inhibitor and a TLR-3 agonist for use as a treatment of solid tumours.

Preferably, the combination of the HIF-1 inhibitor and the TLR-3 agonist is in a pharmaceutical composition.

Still preferably the solid tumour belongs to the group of prostate cancer, breast cancer, colon cancer, lung cancer, renal cancer, metastatic malignant melanoma, brain tumor, bladder cancer and liver cancer.

In a preferred embodiment the tumour is a prostate adenocarcinoma.

Preferably the prostate adenocarcinoma is androgen dependent.

Still preferably the prostate adenocarcinoma is androgen independent.

In the present invention the HIF-1 inhibitor is any known natural, isolated or synthetic HIF-1 inhibitor. It may be in the form of a chemical compound (symthetic or natural) or an interfering nucleic acid sequence targeting *hif-1α* as reported in [6].

In a preferred embodiment the HIF-1 inhibitor is selected from the group of: PX-478, YC-1, echinomycin, polyamides, DJ12, berberine, pseudolaric acid B or bisphenol A.

In the present invention the TLR-3 agonist is any known natural, isolated or synthetic TLR-3 agonist.

In a preferred embodiment the TLR-3 agonist is selected from the group of: Poly(I:C)-LMW, poly I:poly C12U, Poly(A:U) or a dsRNA.

The invention will be now illustrated by means of non-limiting examples referring to the following figures.

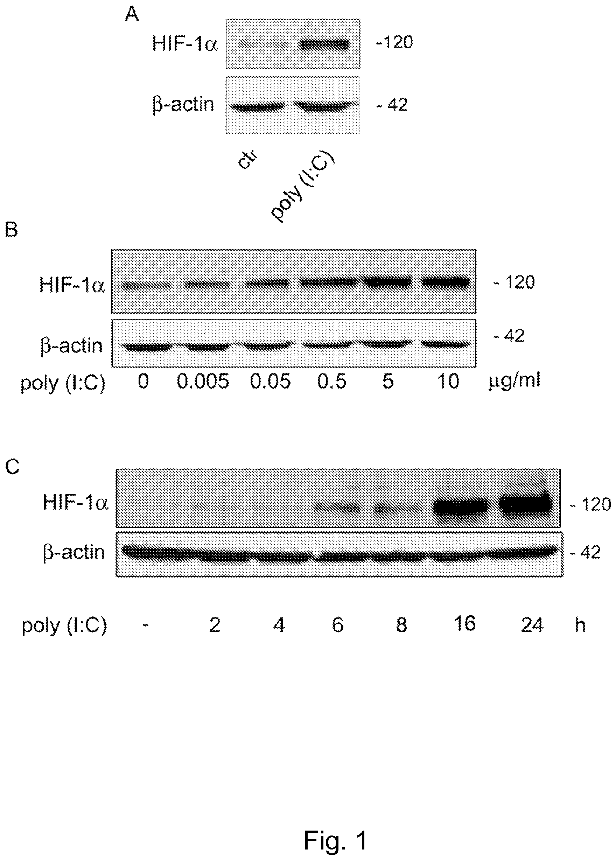

**Fig. 1** Poly(I:C) treatment induces time- and dose-dependent HIF-1α nuclear accumulation in PC3 cells Western blot analysis. (**A**) Cells were treated with poly(I:C) (25 µg/ml) for 6 h and whole cell extracts were analyzed for HIF-1α. **(B)** Cells were treated with the indicated doses of poly(I:C) for 6 h, and nuclear extracts were analyzed for HIF-1α. **(C)** Nuclear extracts from cells treated with 5 µg/ml poly(I:C) for the indicated times. β-actin was used as control for equal amount (30 µg/lane) of protein loaded. Data shown are typical of three separate experiments with similar results. Ctr = control untreated cells.

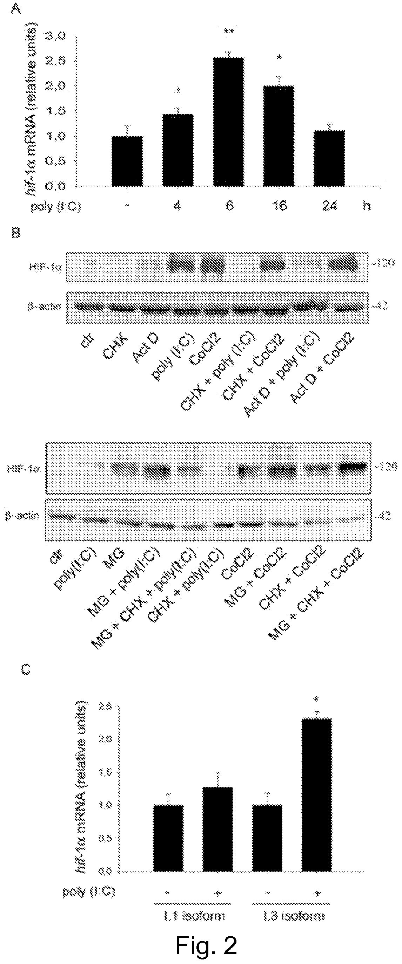

**Fig. 2** Poly(I:C) challenge specifically induces 1.3 isoform of HIF-1α in PC3 cells. (**A**) Cells untreated (Ctr) or treated with 5 µg/ml poly(I:C) for the indicated times were analyzed for *hif-1α* mRNA levels by qRT-PCR. Results represent the mean from three experiments with SD as error bars, *p ≤ 0.05, **p ≤ 0.01. (**B**) Cells were treated with 5 µg/ml poly(I:C) for 5h.45 min. and then with cycloheximide (CHX) (100 µg/ml) or actinomycin D (Act D 2 µg/ml) for 15 min (upper panel). Cells were treated with 5 µg/ml poly(I:C) for 20h.30 min. with or without 5 µg/ml MG-132 for the last 3 hours in the presence or absence of CHX (2 µg/ml) for 30 min. (lower panel). Treatment with 100 µM CoCl₂ for 3 h was used as hypoxic stimulus. HIF-1α protein was detected by Western blot on whole cell lysates. β-actin was used as loading control. (**C**) qRT-PCR with specific primers was used to evaluate the different *hif-1α* isoforms induced after 5 µg/ml poly(I:C) challenge. Data shown in **B** are typical of three separate experiments with similar results. The histogram in **C** represents the mean from three experiments with SD as error bars, *p ≤ 0.05, **p ≤ 0.01.

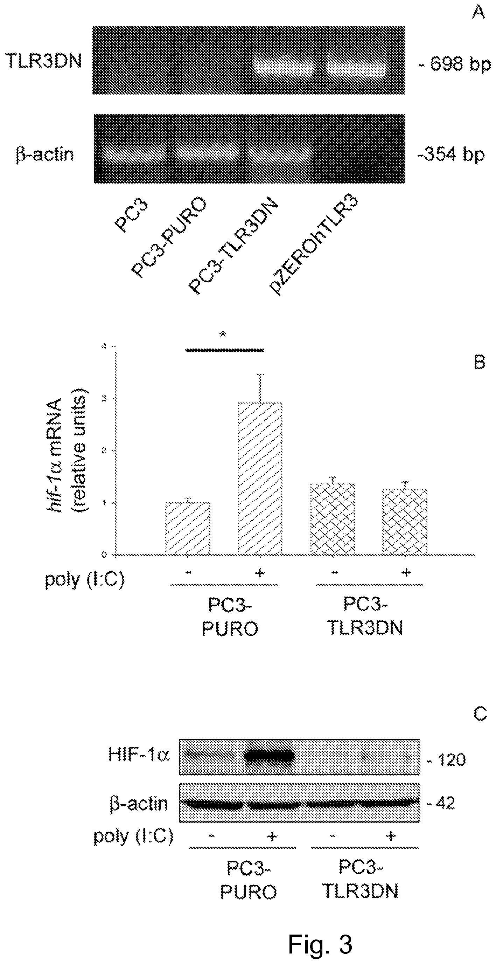

**Fig. 3** Poly(I:C)-induced HIF-1α accumulation is TLR-3 dependent. (**A**) PC3 cells stably transfected with a plasmid containing dominant the negative form of TLR3 (PC3-TLR3DN) or with a plasmid containing only the resistance to puromycin (PC3-PURO) were subjected to total DNA extraction and the presence of the TLR3-DN plasmid was assessed using PCR with specific primers. Non transfected PC3 cells and the plasmid used for the transfection (pZERO-hTLR3) were used as negative and positive control respectively. β-actin was used as control for equal amount of DNA loaded. (**B** and **C**) Both stably transfected cell lines were treated for 6 h with 5 µg/ml poly(I:C) and the increase in *hif-1α* mRNA and nuclear protein accumulation were evaluated by qRT-PCR (**B**) and Western blot analysis (**C**). β-actin was used to normalized both qRT-PCR and Western blot. Data shown in **A** and **C** are typical of three separate experiments with similar results. The histogram in **B** represent the mean ± SD of three independent experiments, *p ≤ 0.05.

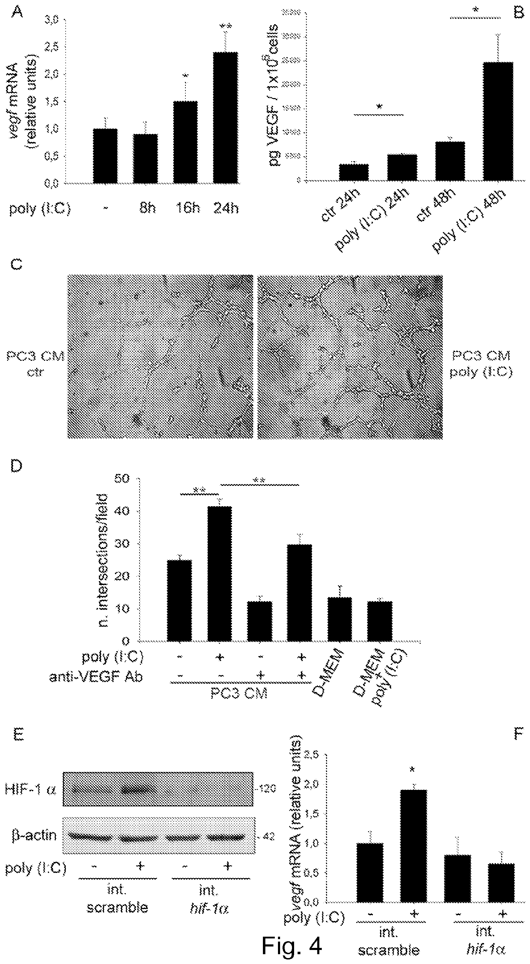

**Fig. 4** Poly(I:C) increases the production of active VEGF through HIF-1α. (**A** and **B**) *vegf* mRNA and protein levels of PC3 cells were evaluated after 5 µg/ml poly(I:C) challenge for the indicated time points using qRT-PCR (**A**) and ELISA assay (**B**), respectively. (**C** and **D**) Formation of capillary-like structures in PC3 cells in conditioned medium (PC3 CM). (**C**) Representative phase contrast microphotographs of capillary-like structures (**D**) and quantitative evaluation of their morphogenesis in HUVEC cultures. HUVEC were exposed to supernatants of PC3 cells untreated (ctr) or treated with poly(I:C) for 48h, in presence or absence of VEGF neutralizing antibodies (anti-VEGF Ab). HUVEC exposed to serum free medium (D-MEM) with or without poly(I:C) for 48 h were used as negative controls. (**E**) Cells were transiently transfected with a vector encoding an RNA interfering *hif-1α* (int. *hif-*1α) or with a vector encoding a scramble RNA sequence (int. scramble), then treated with 5 µg/ml poly(I:C) for 6 h and analyzed for total HIF-1α protein level using Western Blot. β-actin was used as control for equal amount of protein loaded. (**F**) Transfected cells were treated with poly(I:C) for 24 h and *vegf* mRNA expression was evaluated by qRT-PCR. The histograms in **A, B, D,** and **F** represent the mean ± SD of three independent experiments, *p ≤ 0.05, **p ≤ 0.01. Data shown in **E** are typical of three separate experiments with similar results.

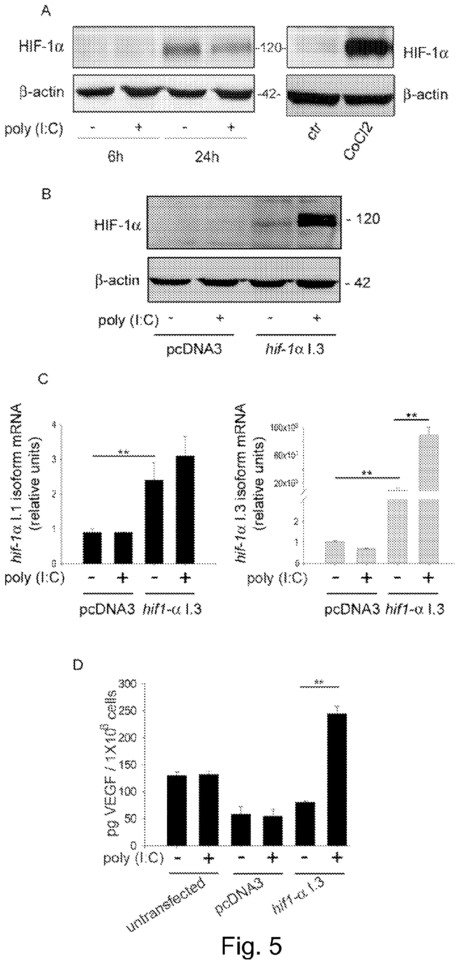

**Fig. 5** Over-expression of I.3 *hif-1α* in LNCaP cells determines poly(I:C)-induced VEGF production. (**A**) LNCaP cells were treated for the indicated times with 25 µg/ml poly(I:C), and Western blot analysis of HIF-1α was performed on nuclear extracts. Cells treated with 100 µM CoCl₂ for 3 h were used as positive control for HIF-1α accumulation. β-actin was used as loading control. (**B, C** and **D**) LNCaP cells were transiently transfected with a plasmid containing the I.3 isoform of HIF-1α (*hif-*1α 1.3) or with a control plasmid (pcDNA3). Cells were then treated with 5 µg/ml poly(I:C) for 6 h and HIF-1α protein levels were analyzed using Western Blot (**B**). Analysis for 1.1 and I.3 isoform of *hif-1α* mRNA was performed by using qRT-PCR (**C**). ELISA assay was used to analyze VEGF concentration in the cell medium (CM) of un-transfected or transfected cells with the indicated plasmids and then treated or left untreated with poly(I:C) (**D**), the histogram represents the mean ± SEM of three independent experiments. **p≤0.01. Data shown in **A** and **B** are typical experiments repeated three times with similar results. Histograms in **C** and **D** represent the mean ± SD of three independent experiments. *p≤ 0.05,**p ≤ 0.01.

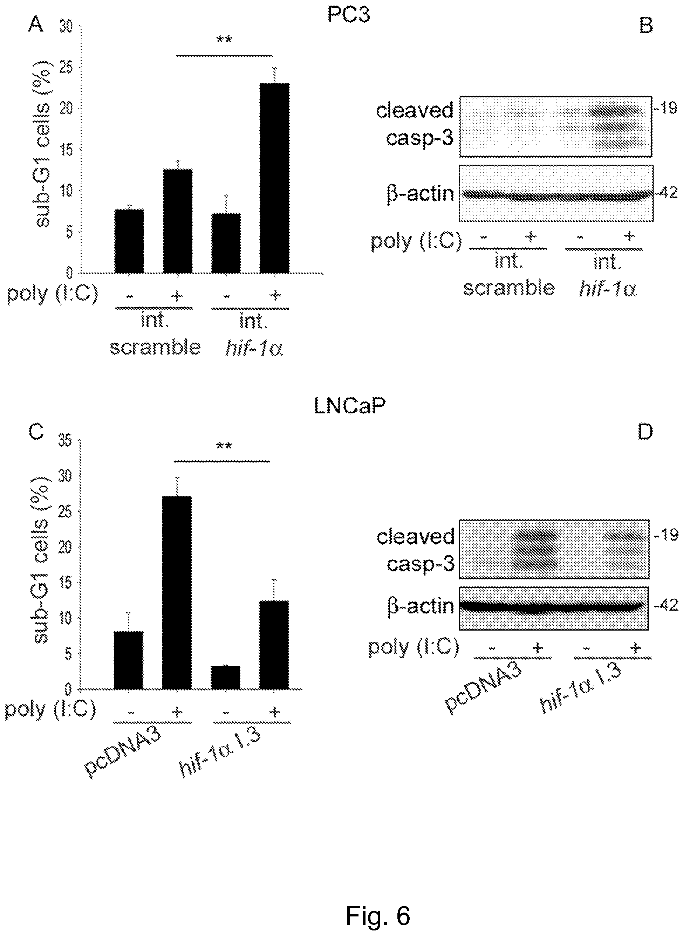

**Fig. 6** Up-regulation of HIF-1α inhibits poly(I:C)-induced apoptosis in LNCaP and PC3 cells. PC3 cells transfected with scramble or *hif-1α* interfering plasmids, treated or not with 5 µg/ml poly(I:C) for 24 h were analyzed for the apoptotic rate using propidium iodide (PI) staining **(A)** and caspase-3 activation **(B).** LNCaP cells overexpressing 1.3 isoform of *hif-1α* were treated with 5 µg/ml poly(I:C) for 24 h and PI staining **(C)** and caspase-3 activation **(D)** were performed to evaluate the apoptotic rate. Histograms in **A** and **C** represent the mean ± SD of three independent experiments. **p≤0.01

### MATERIALS AND METHODS

### Cell lines and reagents

LNCaP and PC3 prostate cancer cells were purchased from ATCC and were grown in DME medium supplemented with 2 mM L-glutamine, 100 IU/ml penicillin-streptomycin and 10% FCS (Sigma-Aldrich, St Louis, MO). For all the experiments, prostate cancer cell lines were serum starved for 18 h and then stimulated with poly(I:C) (InvivoGen, San Diego) in FCS-free medium. Human Umbilical Vein Endothelial Cells (HUVEC) were maintained in EGM®-2 complete medium (Lonza, Basel, Switzerland). Actinomycin D, cycloheximide and CoCl₂ were from Sigma. MG-132 was from Calbiochem-Merk (Damstadt, Germany).

### Western blotting and nuclear extracts preparation

Cell lysates were prepared in cell lysis buffer (Cell Signaling, Danvers, MA). Equal amounts of proteins (40 µg) were subjected to SDS-polyacrylamide gel electrophoresis and transferred onto a nitrocellulose membrane saturated with 5% non-fat dry milk in Tris-buffered saline with 0.1% Tween-20 (T-TBS). Membranes were incubated with primary antibody and subsequently with horseradish peroxidase-conjugated (HRP) secondary antibody for 1 h at room temperature. Membranes were washed with T-TBS and developed using the chemiluminescence system (ECL; Amersham Bioscience, Piscataway, NJ). Antibody anti-HIF-1α was from BD biosciences (San Diego, CA), cleaved caspase-3 (Asp175) from Cell Signalling (Danvers, MA) and anti-β actin from Sigma. Secondary antibodies were HRP-conjugated goat anti-mouse or anti-rabbit (Bio-Rad, Hercules, CA). To perform Western blot of nuclear extracts, cells were washed in ice-cold PBS, scraped and collected in buffer A (10 mM Tris, pH 7.8, 1.5 Mm MgC12, and 10 mM KCl), and kept on ice for 10 min. before Dounce homogenization. Nuclei were then pelleted at 1000 g for 7 min. at 4°C, re-suspended in 150 µl ice-cold lysis buffer and incubated for 30 min. on ice in the presence of proteases inhibitor cocktail (Sigma) then stored at -80°C. Protein concentration was determined by the micro bicinchoninic acid (BCA) method (Pierce, Rockford, IL).

### Transfection assay

One day after plating (1 x 10⁵ cells/ml), cells were transiently transfected with 1 µg of a plasmid encoding 1.3 isoform of *hif-1α* [6] or relative control plasmid (pcDNA3) or with 1 µg of a plasmid containing an interfering sequence targeting *hif-1α* (int.HIF-*1α*) or the relative control (int. scramble) previously described [31]. Cells were transfected for 5 h with lipofectamine LTX/plus (Invitrogen, Carlsbad, CA) according to the manufacturer's protocol. Challenge with poly(I:C) was performed 18 h later.

To obtain stably transfected PC3 cells with non-functional TLR3, cells were transfected with 1 µg TLR3-dominant negative plasmid (TLR3-DN) (pZERO-hTLR3, Invivogen) or with 1 µg plasmid containing only the resistance to puromycin (pPURO), using lipofectamine LTX/plus as described above. 48 h later, the cells were trypsinized, and seeded at different dilutions onto 100-mm plates in complete DMEM supplemented with 2 µg/ml puromycin (Invivogen). After incubation at 37°C for 2 to 3 weeks, individual colonies were isolated using sterile cloning rings, trypsinized and plated onto 12-well plates. The clones obtained were expanded and maintained in DMEM supplemented with 2 µg/ml puromycin.

Total DNA was extracted using TRIZOL Reagent (Invitrogen) according to the manufacturer's protocol and in order to confirm the presence of TLR3DN plasmid in the clones selected semi-quantitative PCR was performed using 1 µg total DNA, specific primers (10 µM) (forward 5'-GAACGTTCTTTTTCGCAACG-3' (SEQ ID No. 2), reverse. 5'-CTCATTGTGCTGGAGGTTCA-3' (SEQ ID No. 3)), and 1.5 U *Taq* DNA polymerase (Invitrogen, Carlsbad, CA). Data were normalized for the expression of β-actin.

### RNA extraction and qRT-PCR analyses

Total RNA was extracted using TRIZOL Reagent (Invitrogen) and used 3 µg for the reverse transcription reaction by using a SuperScript First-strand Synthesis System kit (Invitrogen). 1 µg total cDNA was used for qRT-PCR analysis performed in triplicate for each sample. cDNA was mixed with 0.5 µM of both forward and reverse primers and with 20 µl Master mix (SYBR Green JumpStart; Sigma). Reactions were performed by a 7500 Fast Real-Time PCR System (Applied Biosystems, Carlsbad, CA). Primer sequences: generic *hif-1α*: forward, 5'TGGCCTTGTGAAAAAGGGT3' (SEQ ID No. 4), reverse, 5'TTGATGGGTGAGGAATGGGT3' (SEQ ID No. 5); *vegf* forward 5'CCTTGCTGCTCTACCTCCAC3' (SEQ ID No. 6), reverse 5'TGGTGATGTTGGACTCCTCA3'(SEQ ID No. 7). Amounts of the specific isoform of *hif-1α* were estimated by the comparative Ct method using ribosomal protein L32 mRNA (forward 5'CATCTCCTTCTCGGCATCA3' (SEQ ID No. 8) and reverse 5'AACCCTGTTGTCAATGCCTC3' (SEQ ID No. 9)), 1.3 isoform mRNA (forward 5'TGGTGGTTACTCAGCACTTTTAGA3' (SEQ ID No. 10) and reverse 5'CTCCGACATTGGGAGCTCAT3' (SEQ ID No. 11)) and 1.1 isoform mRNA ( forward 5'TTGCCTTTCCTTCTCTTCTCC3' (SEQ ID No. 12) and reverse 5'TTTCTTTTCGACGTTCAGAACTTAT3' ((SEQ ID No. 13).

### ELISA assay

The level of VEGF was evaluated by ELISA assay (R&D Systems, Minneapolis, MN) according to the manufacturer's protocol and normalized to the number of adherent cells counted at the time of collection.

### In vitro morphogenesis

5x10⁴ HUVEC were seeded in 48-well microtiter plates containing polymerized matrigel and incubated with conditioned media (CM) obtained from either untreated or poly(I:C)-treated cells, or in the presence of specific human VEGF neutralizing antibody (1 µg/ml, R&D Systems). After 6 h, cells were fixed with 4% formaldehyde in PBS for 30 min at RT, washed and stored in PBS at 4°C. The number of cell intersections in 6 random microscopic fields was counted in triplicate wells at 10X original magnification, using the inverted phase contrast microscope Leitz Fluovert (Wetzlar, Germany).

### Apoptosis assay

For propidium iodide (PI) staining, cells were detached with trypsin, washed with cold PBS plus 5% FCS and then fixed in 70% ethanol for 24 h. After washing with PBS, cells were incubated with 1 µg/ml PI for 3 h at 25°C before FACS analysis by a Coulter Epics XL flow cytometer (Beckman Coulter, Fullerton, CA). Cells were considered apoptotic when their DNA content was <2N (sub-G1 cell population). Results were analyzed with Win MDI software (Scripps Research Institute, La Jolla, CA) and presented as percentage of specific apoptosis determined using the following formula: [(% apoptotic cells in experimental sample - % apoptotic cells in control sample)/(100 -% apoptotic cells in control sample) x 100].

### Statistical analysis

Statistical differences were determined either by Student's t-test for paired samples or by one-way analysis of variance followed by Student's t-test with the Bonferroni's correction. p ≤ 0.05 was considered significant. Densitometric analysis were performed using AIDA software.

### RESULTS

### HIF-1α is up-regulated by poly(I:C) in PC3 cells

Since it has been reported that the TLR4, TRL7 or TRL8 agonists induce hypoxic genes in macrophages in normoxic condition [18,19], the authors examined the possibility that TLR3 stimulation could modulate HIF-1α protein in PC3 cells. Cells were treated with the TLR3 agonist, poly(I:C), and levels of HIF-1α determined by Western blot. As shown in Fig. 1A, poly(I:C) induces a marked upregulation of HIF-1α levels in total cell lysates. Since activated HIF-1α rapidly moves into the nucleus, cells were treated with increasing doses of poly(I:C) for 6h and HIF-1α expression evaluated in nuclear extracts. As shown in Fig. 1B, nuclear accumulation of HIF-1α was strongly induced by poly(I:C) in a dose-dependent manner. Time-course analysis showed that an increase of HIF-1α was first observed in the nuclear extracts after 6 h exposure to 5 µg/ml poly(I:C) and maximal induction obtained after 16 h (Fig. 1C). These results demonstrate that in PC3 cells challenge with poly(I:C) induces accumulation of HIF-1α and its translocation to the nucleus.

### HIF-1α accumulation upon poly(I:C) treatment is mostly accounted for by the induction of a specific isoform of HIF-1α mRNA

It is well known that protein stabilization is the predominant mechanism by which HIF-1α protein is increased during hypoxia, while the regulation of HIF-1 under normoxic conditions is less clear [7]. As several papers reported that increased transcription of the *hif-1α* gene accounts for HIF-1α protein induction under normoxic conditions [32], the authors hypothesized that this mechanism could be implicated in the induction of HIF-1α by poly(I:C) in PC3 cells and investigated whether *hif-1α* mRNA was directly up-regulated in a time-course qRT-PCR experiment. A statistically significant increase in the levels of *hif-1α* mRNA was induced within 4 h of poly(I:C) challenge and a further boost was observed after 6h (Fig. 2A). To confirm that the increase in *hif-1α* mRNA is important for elevated protein levels, PC3 cells were treated with poly(I:C) for 6 h, then transcriptional inhibitor actinomycin D (Act D) or protein synthesis inhibitor cycloheximide (CHX) were added and incubated for 15 minutes. Fig. 2B (upper panel) shows that both Act D and CHX completely abolished poly (I:C)-dependent HIF-1α protein induction, whereas, in cells treated with CoCl₂, a chemical hypoxia-mimic that induces stabilization of HIF-1α protein, CHX and Act D did not inhibit HIF-1α protein accumulation, as expected. Indeed, this observation is consistent with previous studies showing that CoCl₂ as well as hypoxia had no effect on HIF-1α protein synthesis but blocked its degradation. On the contrary, the present invention results indicated that induction of HIF-1α protein by poly(I:C) needed de novo synthesis of both hif-1α mRNA and protein in PC3 cells. Next, the authors analyzed whether poly(I:C) enhances HIF-1α protein stability and, to this end, the authors treated PC3 cells with proteasome inhibitor MG-132 to block degradation of HIF-1α. As shown in Fig. 2B (lower panel), in the absence of poly(I:C), MG-132 induced an increase in the level of HIF-1α.

When the MG-132-treated cells were also exposed to poly(I:C), the authors observed a substantial increase of HIF-1α protein level compared to MG-132 alone, while addition of CHX reverted the increase to the level of MG-132 alone, confirming a crucial role of *de novo* synthesis of HIF-1α protein in poly(I:C)-induced HIF-1α accumulation. In contrast, exposure of PC3 cells to CoCl₂ combined with MG-132 resulted in a greater increment of HIF-1α compared to CoCl₂ alone, an increase which was not significantly inhibited by CHX, as expected (Fig. 2B, lower panel). In accordance with this result, the authors did not observe, after poly(I:C) treatment, reactive oxygen species (ROS) accumulation (results not shown), which has been implicated in TLR-mediated HIF-1α stabilization due to down-regulation of prolyl hydroxylase [35b].

Since the authors recently described a novel mRNA isoform 1.3 of HIF-1α that is up-regulated in activated human T lymphocytes, they investigated the levels of different *hif-α* isoforms in PC3 cells and their regulation through TLR3 . Since the primers used for the above qRT-PCR amplify both 1.1 and 1.3 *hif-1α* isofoms, the authors used isoform-specific primers in order to study which isoform of *hif-1α* was induced by poly(I:C). As shown in Fig. 2C, 1.1 isoform of *hif-1α* gene, canonically activated by hypoxia, is not affected by poly(I:C) challenge, whereas 1.3 isoform mRNA is up-regulated after 6h of treatment at the same degree observed in qRT-PCR for total *hif-1α* mRNA shown in Fig. 2A.

### Poly(I:C)-induced HIF-1 α accumulation is TLR-3 dependent

Different molecules besides TLR3 are involved in dsRNA recognition, since poly(I:C) is able to activate also different pathways mediated by cytosolic sensors [33,34]. To determine whether the observed HIF-1α accumulation was directly dependent on TLR3 stimulation, the authors produced a PC3 cell line (PC3-TLR3DN) stably transfected with a dominant negative form of TLR3 (pZERO-hTLR3) and another (PC3-PURO) with a plasmid containing solely the resistance to puromycin. DNA analysis with specific primers showed the presence of the pZERO-hTLR3 plasmid only in the PC3-TLR3DN cells (Fig. 3A). Hence, both transfected clones were treated with poly(I:C) and the increase in *hif-1α* mRNA and nuclear protein accumulation were evaluated by qRT-PCR and Western Blot, respectively. As shown in Fig. 3B and C, the expression of TLR3-DN completely inhibits poly(I:C)-induced HIF-1α transcription and nuclear protein accumulation, while HIF-1α up-regulation was confirmed in poly(I:C)-treated PC3-PURO control cells. These data demonstrate the key role of TLR3 in mediating the induction of HIF-1α triggered by poly(I:C).

### Poly(I:C) induces HIF-1α-mediated VEGF transcription and secretion

In order to test whether the enhanced HIF-1α protein level induced by poly(I:C) is paralleled by an increase in its transcriptional activity, PC3 cells were stimulated with poly(I:C) for different lengths of time and transcription of *vegf,* a known HIF-1 target gene, was analyzed by qRTPCR. VEGF mRNA levels were significantly enhanced in PC3 cells following 16 and 24 h poly(I:C) treatment (Fig. 4A). Using ELISA assay, the authors also evaluated the amount of VEGF secreted by poly(I:C)-treated PC3 cells, in conditioned media (CM) collected at various time points during poly(I:C) challenge. The kinetics of VEGF accumulation is subsequent to the induction of *vegf* mRNA, with a significant increase of VEGF protein in PC3-CM already detectable after 24h challenge and strong accumulation after 48h (Fig. 4B). In order to validate the angiogenic potential of VEGF secreted under poly(I:C) challenge, the tubulogenic activity of CM was tested on endothelial cell line HUVEC. As shown in Fig. 4C and D, poly(I:C)-treated PC3 cell CM was found to stimulate the formation of more capillary-like structures than the CM from control PC3 cells. Moreover, anti-VEGF antibody significantly reverted this pro-angiogenic function, confirming that VEGF is the main mediator of poly(I:C)-induced *in vitro* angiogenesis (Fig. 4D). No direct effect of poly(I:C) on tubulogenesis was observed (Fig. 4D). To clearly demonstrate the involvement of HIF-1α in the stimulation of VEGF secretion, PC3 cells were transiently transfected with a vector encoding an RNA interference targeting *hif-1α* (int.HIF-1α) or with a vector encoding a scramble RNA interference sequence (int.scramble). At first the authors verified that poly(I:C)-induced HIF-1α protein was significantly reduced in cells with int.HIF-1α (Fig. 4E). Transfected cells were treated with poly(I:C) for 24 h and *vegf* expression was analyzed by qRT- PCR. The RNA interfering against *hif-1α,* but not scramble sequence, significantly inhibited poly(I:C)-induced *vegf* transcription (Fig. 4F) demonstrating that poly(I:C) can induce VEGF production by increasing HIF-1α protein expression, and the formation of a transcriptionally active HIF-1 complex.

### Poly(I:C) induces VEGF secretion in LNCaP cells over-expressing I.3 hif-1α isoform

It has been published that LNCaP cells have the lowest HIF-1α levels among human and rat PCa cell lines, suggesting that HIF-1α expression may increase in cancer progression [35]. Therefore, the authors studied whether TLR3 stimulation can induce HIF-1α nuclear accumulation also in LNCaP cells and found that poly(I:C) challenge failed to induce nuclear accumulation of HIF-1α (Fig. 5A). Total lysates of LNCaP cells treated with CoCl₂ were used as positive control of HIF-1α accumulation. The poly(I:C)-independent weak accumulation of HIF-1α at 24h is an effect of serum-starvation, as previously described [36]. To study the effects of 1.3 *hif-*1α over-expression in LNCaP cells treated or not with poly(I:C), the authors transfected this cell line with a plasmid containing 1.3 isoform of *hif-1a* (*hif-1a* I.3) and treated the cells with poly(I:C). Over-expression of *hif-1a* 1.3 isoform in LNCaP cells induced a slight increase of HIF-1α protein, strongly raised upon poly(I:C) treatment (Fig. 5B). The mechanism of poly(I:C)-induced HIF-1α accumulation in transfected LNCaP cells, was further investigated by qRT-PCR and revealed that the transfection with *hif-1a* 1.3 induces a 2000-fold accumulation of this mRNA compared to untransfected LNCaP cells and that poly(I:C) challenge induces a further 5-fold accumulation above the basal levels of the transfected cells (Fig. 5C right panel). The left panel of Fig. 5C shows that 1.3 isoform transfection causes 1.1 *hif-1a* mRNA to increase by approximately 3-fold over the basal levels and that poly(I:C) fails to induce additional accumulation. These data confirm the induction of the specific 1.3 isoform of *hif-1α* mRNA by poly(I:C), suggesting an important role of the basal levels of this isoform in this process. To understand if poly(I:C)-induced HIF-1α protein was functional in *hif-1α* 1.3 transfected LNCaP cells, the authors checked for VEGF accumulation in LNCaP CM by ELISA assay. Fig. 5D shows that poly(I:C) fails to induce VEGF accumulation in LNCaP cells transfected with pcDNA3 control plasmid, in accordance with the lack of HIF-1α accumulation shown in Fig. 5A. In the CM of the cells transfected with the 1.3 plasmid the accumulation of VEGF is only weak respect to the medium of the cells transfected with the control plasmid but a very strong accumulation of VEGF is detectable in the medium of the cells transfected with *hif-1α* 1.3 and then treated with poly(I:C) (Fig. 5D).

### HIF-1α levels regulate poly(I:C)-induced apoptosis in PC3 and LNCaP cells

So far the authors have demonstrated that, unlike PC3 cells, LNCaP cells fail to undergo HIF-1α increase upon TLR3 stimulation. Given that previous papers reported an anti-apoptotic effect for HIF-1α [37] and given the low sensitivity of PC3 cells to poly(I:C)-induced apoptosis [25], the authors hypothesized a role of HIF-1α in their resistance to this apoptotic stimulus. To test this hypothesis, PC3 cells were transfected with int.HIF-1*α* or with int.scramble, treated with poly(I:C) and apoptosis induction was analyzed. Propidium iodide (PI) staining revealed that PC3 cells transfected with int.HIF-1*α* were more susceptible to poly(I:C)-induced apoptosis than the cells transfected with int.scramble (Fig. 6A). Moreover Western blot for activated form of caspase-3 showed that induction of this apoptotic effector is stronger in cells transfected with int.*hif-1α* than those transfected with int.scramble confirming the importance of HIF-1α in regulation of apoptotic susceptibility (Fig. 6B).

To confirm the anti-apoptotic function of the poly(I:C)-induced HIF-1α protein observed in PC3 cells, the authors transfected LNCaP cells with the plasmid containing 1.3 *hif-1α.* Transfected LNCaP cells were treated with poly(I:C) and their apoptotic rate was evaluated by flow cytometry. Fig. 6C shows that the sub-Gl population represents 12% of I.3 *hif-1α* transfected cells after poly(I:C) treatment, whereas this population significantly increases (up to about 25%) in cells transfected with the control plasmid and treated with poly(I:C). The authors analyzed caspase-3 activation also in transfected LNCaP cells treated or not with poly(I:C) and observed that activation of caspase-3 was lower in the cells transfected with 1.3 *hif-1α* than in the cells transfected with the control plasmid (Fig. 6D).

### DISCUSSION

Non-hypoxic stimuli such as LPS, IL1-β, IGF, TNFα, usually associated with inflammatory conditions, have been described to induce HIF-1α protein accumulation and transcription of HIF-1 target genes in several cellular models [7]. The stimulation of Toll-like receptors by microbial components may represent the signal promoting a pro-inflammatory environment that enhances tumour growth and chemoresistance. In the present invention, the authors report that challenge of TLR3 with poly(I:C) can increase HIF-1α mRNA and HIF-1α protein nuclear translocation in the highly aggressive prostate cancer cell line PC3. A very recent paper reported that transfected poly(I:C) induces HIF-1α in a glioblastoma cell line, suggesting a role of this transcription factor in the response to viral infection [38]. In the present invention, the authors extend to prostate cancer cells the involvement of HIF-1α inpoly(I:C) response and for the first time demonstrate a key role of TLR3 in poly(I:C)-induced HIF-1α activation, using a TLR3-dominant negative-stably transfected PC3 cell line. In fact, while transfected poly(I:C) mimics intracellular dsRNA generated during viral replication, extra-cellular administration of poly(I:C) involves TLR3 activation and mimics viral infection through the pinocytosis of dsRNA released by lysed, infected cells. TLR agonists are under clinical trial for the treatment of various carcinoma and even a GMP-grade synthetic analogue of poly(I:C) has been proposed as adjuvant for cancer immunotherapy [39] but this strategy is currently discussed, as several papers report opposite pro- or anti-tumour response after TLR stimulation [40,41]. The authors recently described that poly(I:C) induces a strong TLR3-mediated apoptotic response in LNCaP cells, whereas PC3 cells appear weakly sensitive to this stimulus [25]. The lower sensitivity of PC3 cells to poly(I:C)-induced apoptosis compared to LNCaP cells led the authors to hypothesize that, in the more aggressive PC3 cell line, poly(I:C) challenge may activate genes inducing an anti-apoptotic response.

Resistance to apoptosis is linked to HIF-1α function in prostate cancer [36,42]. Using an RNA interference approach, the authors observed a significant increase of apoptosis in HIF-1α knock-down PC3 cells demonstrating a direct role of HIF-1α in the protection of PC3 cells against poly(I:C)-induced apoptosis.

Beside HIF-1α-mediated anti-apoptotic effect, the authors observed also a consistent increase in poly(I:C)-induced VEGF production. VEGF is one of the principal HIF-1 target genes considered the main factor responsible for the induction of neo-vascularization in hypoxic tumour sites, favouring cancer progression [43]. The ability of poly(I:C) to induce TLR3-dependent VEGF secretion has been recently reported in a mesothelioma cell line [44] but the mechanism of induction was not described. In the present invention, using a plasmid containing an interference sequence for HIF-1α, the authors demonstrate that poly(I:C)-induced HIF-1α activation is responsible for VEGF production in PC3 cells, revealing a new pro-tumour face of TLR3 signaling in prostate cancer.

HIF-1α stabilization is the key event in the induction of HIF-1 complex in hypoxic conditions, but its activation mechanism in normoxic condition is still not clear, probably depending on the nature of the stimulus and on the cell types. It was recently shown both in mouse and human that triggering TCR on T-cells [6,17,18] and TLR4 on macrophages [20] leads to specific up-regulation of alternatively spliced mRNA isoforms of HIF-1α, which contain an alternative first exon. In the present invention, the authors investigated for the first time the levels of different *hif-α* isoforms in tumour cells and report that activation of HIF-1 complex after poly(I:C) challenge in PC3 cells is mainly dependent on the transcription of the HIF-1α 1.3 isoform.

There is accumulating evidence that the main mechanism implicated in the non-hypoxic induction of HIF-1α is an increase in HIF-1α protein translation, and that the rate of degradation of HIF-1α remains high [7]. As for the mechanisms underlying TLR3-mediated HIF-1α up-regulation, the authors demonstrated by pharmacological inhibitors that HIF-1α-increase in PC3 requires new transcription and *de novo* protein synthesis, showing for the first time that poly(I:C) enhances HIF-1α expression through a translation-dependent mechanism rather than inhibition of protein degradation.

Regarding the different mRNA isoforms of HIF-1α, it has been initially shown that in mice HIF-1α is encoded by two alternative isoforms, which are expressed form different promoters and utilizes two distinct exons: 1.1 or 1.2 [17;48b]. The 1.1 isoform, which is analogous to 1.3 human isoform described in the present invention, was expressed in tissue-specific manner and was strongly induced by activation, while 1.2 isoform (analogue of the human 1.1 isoform) demonstrated ubiquitous pattern of expression and was present in all analyzed tissues. Studies of the murine and human HIF-1α promoters [48b;48c] revealed that the ubiquitous isoform is expressed continuously from the housekeeping-type CpG island-rich promoter. On the other hand murine 1.1 isoform and corresponding human 1.3 isoform were shown to be dramatically up regulated by activating stimuli in T lymphocytes [6;17] possibly due to the presence of a number of activation response binding sites for transcriptional factors in the promoter [48b]. Thus, it was expected that the HIF-1α isoform up regulated following TLR3 activation was the 1.3, but not the ubiquitous 1.1 in human cells.

Likely, TLR3 stimulation induces a factor(s) enhancing HIF-1α protein translation and/or stabilization so that the balance between synthesis and degradation is shifted towards accumulation of normoxic HIF-1α.

A previous paper reported amplification of HIF-1α gene in PC3 cells resulting in higher basal level of HIF-1α protein present in the nucleus of these cells with respect to other prostate cancer cell lines such as LNCaP [35]. Moreover, the apoptotic signalling mechanism triggered by poly(I:C) in LNCaP cells is mediated by PKC-α [27b] without increasing HIF-1α, whereas PKC-alpha appears to play a role in HIF-1α accumulation in PC3 cells (data not shown), indicating complex transduction pathways evoked by poly(I:C) in prostate cancer cell lines. In view of these different features of the two cell lines, a common mechanism of HIF-1α activation following poly(I:C) stimulation is highly unlikely. Accordingly, our data show that poly(I:C) induced up-regulation of endogenous HIF-1α in PC3 cells, but not in LNCaP cells.

Surprisingly, transfection of the 1.3 HIF-1α isoform in LNCaP cells enhanced about 200 fold HIF-1α basal mRNA, only slightly increased HIF-1α protein, failing to induce VEGF secretion. Interestingly, in LNCaP cells over-expressing of exogenous HIF-1α 1.3, stimulation with poly(I:C) strongly increases HIF-1α protein, suggesting that TLR3 activation is capable of counteracting the mechanisms that can basally control HIF-1α protein accumulation. The possibility that the effects of this forced overexpression may partially depend on an artificial responsiveness of the exogenous promoter cannot be ruled out.

Noteworthy, poly(I:C) challenge of LNCaP cells over-expressing HIF-1α leads to strong induction of HIF-1α and VEGF proteins paralleled by the capability to resist to poly(I:C)-induced apoptosis. These results strongly indicate that a high expression of I.3 HIF-1α is essential for poly(I:C)-induced HIF-1α accumulation and its target gene activation, implying the existence of an amplification loop. Accordingly, a previous paper reported the presence of an hypoxia responsive element (HRE) on *hif-*1α promoter [45]. Therefore, the deregulation of HIF-1α basal levels appears to be crucial in controlling the mechanisms involved in cancer progression.

HIF-1 has become an important therapeutic target in solid tumours and efficient drugs capable of decreasing HIF-1 are currently under development [46-48]. Moreover, activation of hypoxic genes in prostate, breast, and ovarian cancers as predictor of adverse clinical outcome has been reported, suggesting that HIF-1 complex represents a key transcription factor in endocrine tumours [8]. In particular, HIF-1α/β and HIF-2α, together with abnormal estrogen receptor-β and eNOS, have been recently described to drive prostate cancer towards a more aggressive phenotype [49].

In concluding, the present invention provides the first evidence showing a correlation between HIF-1α 1.3 isoform expression and ability of TLR3 to regulate apoptosis and induce VEGF production in human tumour cell lines. The combination of and is therefore a valid targeted therapeutic strategy for the treatment of prostate cancer.

### REFERENCES

[1] Pugh,C.W., et al. (1991) Proc.Natl.Acad.Sci.U.S.A, 88, 10553-10557.
[2] Semenza,G.L., et al. (1991) Proc.Natl.Acad.Sci.U.S.A, 88, 5680-5684.
[3] Wenger,R.H., Stiehl,D.P., and Camenisch,G. (2005) Sci.STKE., 2005, re12.
[4] Brahimi-Horn,M.C. and Pouyssegur,J. (2009) J.Cell Sci., 122, 1055-1057.
[5] Depping,R., et al. (2004) Biol.Reprod., 71, 331-339.
[6] Lukashev,D. and Sitkovsky,M. (2008) Hum.Immunol., 69, 421-425.
[7] Dery,M.A., et al. (2005) Int.J.Biochem.Cell Biol., 37, 535-540.
[7b] Hoffinann,A.C., et al. (2008) Neoplasia, 10, 674-679.
[8] Kimbro,K.S. and Simons,J.W. (2006) Endocr.Relat Cancer, 13, 739-749.
[9] Zhong,H., et al. (2004) Cancer Detect.Prev., 28, 88-93.
[10] Zhong,H., et al. (1999) Cancer Res., 59, 5830-5835.
[11] Gomella,L.G., Johannes,J., and Trabulsi,E.J. (2009) Urology, 73, S28-S35.
[12] Brune,B. and Zhou,J. (2003) Curr.Med.Chem., 10, 845-855.
[13] Gorlach,A. and Kietzmann,T. (2007) Methods Enzymol., 435, 421-446.
[14] Gerber,S.A. and Pober,J.S. (2008) J.Immunol., 181, 1052-1062.
[15] Jung,Y., et al. (2003) Biochem.J., 370, 1011-1017.
[16] Zelzer,E., et al. (1998) EMBO J., 17, 5085-5094.
[17] Lukashev,D., et al. (2001) J.Biol.Chem., 276, 48754-48763.
[18] Blouin,C.C., et al. (2004) Blood, 103, 1124-1130.
[19] Nicholas,S.A. and Sumbayev,V.V. (2009) Cell Res., 19, 973-983.
[20] Ramanathan,M., et al. (2009) J.Leukoc.Biol., 86, 681-689.
[21] Akira,S., Uematsu,S., and Takeuchi,O. (2006) Cell, 124, 783-801.
[22] Iwasaki,A. and Medzhitov,R. (2004) Nat.Immunol., 5, 987-995.
[23] Huang,B., et al. (2005) Cancer Res., 65, 5009-5014.
[24] Kelly,M.G., et al. (2006) Cancer Res., 66, 3859-3868.
[25] Paone,A., et al. (2008) Carcinogenesis, 29, 1334-1342.
[26] Salaun,B., et al. (2006) J.Immunol., 176, 4894-4901.
[27] Salaun,B., et al. (2007) Clin.Cancer Res., 13, 4565-4574.
[27b] Paone,A., et al. (2008) Carcinogenesis, 29, 1334-1342.
[28] Chen,N., et al. (2009) J.Biol.Chem., 284, 10004-10012.
[29] Peng,X.H., et al. (2006) J.Biol.Chem., 281, 25903-25914.
[30] Piret,J.P., et al. (2005) J.Biol.Chem., 280, 9336-9344.
[31] Bonello,S., et al. (2007) Arterioscler.Thromb.Vasc.Biol., 27, 755-761.
[32] Gorlach,A. (2009) Curr.Pharm.Des, 15, 3844-3852.
[33] Clemens,M.J., et al. (1993) J.Interferon Res., 13, 241.
[34] Yoneyama,M., et al. (2004) Nat.Immunol., 5, 730-737.
[35] Zhong,H., et al. (1998) Cancer Res., 58, 5280-5284.
[35b] Nicholas,S.A. and Sumbayev,V.V. (2010) Immunol. Cell Biol., 88, 180-186.
[36] Thomas,R. and Kim,M.H. (2008) Prostate, 68, 1405-1415.
[37] Greijer,A.E. and van der,W.E. (2004) J.Clin.Pathol., 57, 1009-1014.
[38] Hong,S.W., et al. (2009) Mol.Cells, 27, 243-250.
[39] Jasani,B., et al. (2009) Vaccine, 27, 3401-3404.
[40] Conroy,H., et al. (2008) Oncogene, 27, 168-180.
[41] Wolska,A., et al. (2009) Cell Mol.Biol.Lett., 14, 248-272.
[42] Zhou,J., et al. (2006) Cancer Lett., 237, 10-21.
[43] Ferrara,N. (2002) Nat.Rev.Cancer, 2, 795-803.
[44] Wornle,M., et al. (2009) Cell Biol.Int., 33, 180-186.
[45] Minet,E., et al. (1999) Biochem.Biophys.Res.Commun., 261, 534-540.
[46] Mabjeesh,N.J., et al. (2003) Cancer Cell, 3, 363-375.
[47] Narita,T., et al. (2009) Clin.Cancer Res., 15, 6128-6136.
[48] Guan,Y., et al. (2009) Mol.Ther.*.*
[48b] Wenger, RH. et al., (1998) Blood, 91, 3471-3480.
[48c] Iyer, NV. et al., (1998) Genomics, 52, 159-165.
[49] Nanni,S., et al. (2009) J.Clin.Invest, 119, 1093-1108.
[50] Onnis et al. (2009) J. of cell. and mol. med., 13, 2780-2786.
[51] Melillo et al. (2007) Cancer and metastasis reviews, 26, 341-352.

## Claims

1. A combination of a HIF-1 inhibitor and a TLR-3 agonist for use as a treatment of solid tumours.

2. The combination of a HIF-1 inhibitor and a TLR-3 agonist for use according to claim 1 being in a pharmaceutical composition.

3. The combination for use according to claim 1 or 2 wherein the solid tumour belongs to the group of prostate cancer, breast cancer, colon cancer, lung cancer, renal cancer, metastatic malignant melanoma, brain tumor, bladder cancer and liver cancer.

4. The combination for use according to claim 1 or 2 wherein the tumour is a prostate adenocarcinoma.

5. The combination for use according to claim 4 wherein the prostate adenocarcinoma is androgen dependent.

6. The combination for use according to claim 4 wherein the prostate adenocarcinoma is androgen independent.

7. The combination for use according to any one of previous claims wherein the HIF-1 inhibitor is selected from the group of: PX-478, YC-1, echinomycin, polyamides, DJ12, berberine, pseudolaric acid B or bisphenol A.

8. The combination for use according to any one of previous claims wherein the TLR-3 agonist is selected from the group of: Poly(I:C)-LMW, poly I:poly C12U, Poly(A:U) or a dsRNA.

## Patentansprüche

1. Kombination eines HIF-Inhibitors und eines TLR-3-Agonisten zur Verwendung als Behandlung solider Tumore.

2. Kombination eines HIF-Inhibitors und eines TLR-3-Agonisten zur Verwendung nach Anspruch 1, wobei sie eine pharmazeutische Zusammensetzung ist.

3. Kombination zur Verwendung nach Anspruch 1 oder 2, wobei der solide Tumor zu der Gruppe von Prostatakrebs, Brustkrebs, Kolonkrebs, Lungenkrebs, Nierenkrebs, metastatisch bösartigem Melanom, Gehirntumor, Blasenkrebs und Leberkrebs gehört.

4. Kombination zur Verwendung nach Anspruch 1 oder 2, wobei der Tumor ein Prostataadenokarzinom ist.

5. Kombination zur Verwendung nach Anspruch 4, wobei das Prostataadenokarzinom androgenabhängig ist.

6. Kombination zur Verwendung nach Anspruch 4, wobei das Prostataadenokarzinom androgenunabhängig ist.

7. Kombination zur Verwendung nach irgendeinem der vorhergehenden Ansprüche, wobei der HIF-Inhibitor aus der Gruppe ausgewählt wird von: PX-478, YC-1, Echinomycin, Polyamiden, DJ12, Berberin, Pseudolarinsäure B oder Bisphenol A.

8. Kombination zur Verwendung nach irgendeinem der vorhergehenden Ansprüche, wobei der TLR-3-Agonist aus der Gruppe ausgewählt wird von: Poly(I:C)-LMW, Poly I:Poly C12U, Poly(A:U) oder einer dsRNA.

## Revendications

1. Combinaison d'un inhibiteur de HIF-1 et d'un agoniste de TLR-3 pour son utilisation en tant que traitement de tumeurs solides.

2. Combinaison d'un inhibiteur de HIF-1 et d'un agoniste de TLR-3 pour son utilisation selon la revendication 1 étant dans une composition pharmaceutique.

3. Combinaison pour son utilisation selon la revendication 1 ou 2 dans laquelle la tumeur solide appartient au groupe du cancer de la prostate, du cancer du sein, du cancer du côlon, du cancer du poumon, du cancer du rein, d'un mélanome malin métastatique, d'une tumeur cérébrale, du cancer de la vessie et du cancer du foie.

4. Combinaison pour son utilisation selon la revendication 1 ou 2 dans laquelle la tumeur est un adénocarcinome de la prostate.

5. Combinaison pour son utilisation selon la revendication 4 dans laquelle l'adénocarcinome de la prostate est androgéno-dépendant.

6. Combinaison pour son utilisation selon la revendication 4 dans laquelle l'adénocarcinome est androgéno-indépendant.

7. Combinaison pour son utilisation selon l'une quelconque des revendications précédentes dans laquelle l'inhibiteur de HIF-1 est sélectionné dans le groupe suivant : PX-478, YC-1, échinomycine, polyamides, DJ12, berbérine, acide pseudolarique B ou bisphénol A.

8. Combinaison pour son utilisation selon l'une quelconque des revendications précédentes dans laquelle l'agoniste de TLR-3 est sélectionné dans le groupe suivant : Poly(I:C)-LMW, poly I:poly C12U, Poly(A:U) ou un ARNdb.
